# EUROPEAN PATENT APPLICATION

(11) **EP 4 424 361 A1**
(43) Date of publication of application: **04.09.2024**
(21) Application number: 22887138.0
(22) Date of filing: 27.10.2022
(51) Int. Cl.: A61N 5/06

(54) **METHOD FOR TREATING DISORDERS WITH PHOTOSTIMULATION AND INSTRUMENT USED IN SAID METHOD**

(30) Priority: 27.10.2021 JP 2021175619
(71) Applicant: Tsubota Laboratory, Inc., Tokyo 160-0016 (JP)
(72) Inventor: HAYANO Motoshi, Tokyo 160-0016 (JP); TSUBOTA Kazuo, Tokyo 160-0016 (JP)
(74) Representative: Plasseraud IP
(86) International application number: PCT/JP2022/040211
(87) International publication number: WO 2023/074813

(57) **Abstract**

A purpose of the present invention is to provide a method of treatment of a disorder by light stimulation in which light of a specific wavelength such as violet light is irradiated at a specific blinking frequency, and an apparatus used for the same. The present invention treats and/or prevents a disorder by promoting survival, proliferation, and differentiation of oligodendrocyte precursor cells or oligodendrocytes, regenerating or increasing myelin, activating nerves, and activating immunity by irradiating light of a specific wavelength such as violet light to a living body.

## Description

### Technical Field

The present invention relates to a method of treatment of disorder by light stimulation and an apparatus used for the same, and more particularly, relates to a method of treatment of a disorder by light stimulation in which light of a specific wavelength such as violet light is irradiated at a specific blinking frequency, and an apparatus used for the same.

### Background Art

Effects of light on a human body have been studied from various perspectives in recent years, and reported on the basis of new findings. For instance, a fact that circadian rhythm is improved by bathing in the sunlight (Non-Patent Document 1), facts such as, light irradiated from a display such as a liquid-crystal display in which LED (light emitting diode) lighting and an LED is used as a backlight, have a substantial effect on body and mind (Non-Patent Document 2), and violet light prevents myopia and suppresses development of myopia (Patent Document 1) have been reported. Recently, in particular, inventors of the present invention have submitted an interesting report about effects of violet light on eyes, and in Patent Document 1 and Non-Patent Document 3 for example, it has been proposed that light of a specific wavelength is effective in preventing myopia and suppressing myopia, and with myopic population still on the rise globally in recent years, high expectations are placed.

Moreover, research and development of various therapeutic techniques has also been active, and research and development of therapeutic techniques with reduced burden on body, particularly by not using medication or reducing the use thereof, has been carried out. Disorders to be treated, besides demyelination disorder, include for example, amyotrophic lateral sclerosis (ALS), degenerative neurological disorders such as Alzheimer disease and spinal damage, tumors such as brain tumor (glioma), and central neurological disorders such as cerebral infarction.

Demyelination disorder refers to a disorder caused due to degeneration and loss of myelin sheath of nerve fiber. Multiple sclerosis (MS) can be cited as an example of demyelination disorder. Multiple sclerosis is a chronic inflammatory disorder of central nervous system characterized by damage of myelin, and a functional disorder of brain, optic nerve, and spinal cord. Although research and development of a method of treatment of demyelination disorder including multiple sclerosis has been carried out actively, an effective treatment method has not been established, and further therapeutic options are being sought.

Oligodendrocytes have been known to be associated with various disorders, and Non-Patent Document 4, for instance, indicates that oligodendrocytes decrease with aging. Non-Patent Document 5 indicates that oligodendrocytes decrease in Alzheimer disease. Non-Patent Document 6 indicates that oligodendrocyte differentiation is significant for recovery from cerebral infarction. Non-Patent Document 7 indicates that oligodendrocyte differentiation is significant for recovery from spinal damage.

Fibroblast growth factor 21 (FGF21) is a pleiotropic hormone which has been considered as a main regulatory factor of energy homeostasis. Although FGF21 is secreted mainly from liver, it has been known that it might as well is developed in skeletal muscles. It has been known that FGF21 signaling to glutamatergic neurons in hypothalamic area induces decrease in a sugar ingestion amount of a mouse, and is necessary for changing the liking of sweet taste. Other studies indicate that FGF21 activates PGC-1α and improves an effectiveness of mitochondria in human dopaminergic neurons, and suggest that there is a possibility of FGF21 playing a role in PD and survival capability of dopaminergic neurons. Peroxisome proliferator-activated receptor γ coactivator-1α (PGC-1α) is a main regulatory factor of mitochondrial function and biosynthesis, and was proposed as a therapeutical target of PD. Non-Patent Documents 8 to 12 disclose functions of FGF21.

### Citation List

### Non-patent Documents

Non-Patent Document 1: Anti-aging Medicine by Megumi Hatori and Kazuo Tsubota, Japanese Society of Anti-aging Medicine MagazineVol. 11, No. 3, 065 (385) -072 (392), (2015)
Non-Patent Document 2: `Blue Light Hazard' by Kazuo Tsubota, Published by Shueisha on November 20, 2013
Non-Patent Document 3: Hidemasa Torii et al., EBioMedicine, `DOI: http://dx.doi.org/10.1016/j.ebiom.2016.12.007'.
Non-Patent Document 4: Nat Neurosci. 2018 May; 21(5): 683-695.
Non-Patent Document 5: Neuron. 2021 Jul 21; 109(14): 2292-2307. e5.
Non-Patent Document 6: Aging Dis. 2021 Dec 1; 12(8): 2096-2112.
Non-Patent Document 7: Front Cell Neurosci. 2021 Jan 11; 14: 619707.
Non-Patent Document 8: J Clin Invest. 2017 Sep 1; 127(9): 3496-3509.
Non-Patent Document 9: Cell Rep. 2022 Aug 23; 40(8): 111239.
Non-Patent Document 10: Nature Communications volume 13, Article number: 1897(2022).
Non-Patent Document 11: Nature, 2010 Jun 10; 465(7299): 783-7.
Non-Patent Document 12: Eur J Neurosci. 2021 Jan; 53(1); 140-150.

### Patent Documents

Patent Document 1: WO2015/186723A1

### Summary of the Invention

### Problems to be Solved by the Invention

The inventors of the present invention discovered that irradiation of light of a specific wavelength, particularly violet light (visible light rays in a region of 360 nm to 400 nm), at a blinking frequency accelerates survival, proliferation, and differentiation of oligodendrocyte precursor cells and oligodendrocytes, accelerates regeneration and an increase in myelin, activates nerves, and activates immunity. The present invention is based on such discovery, and an object thereof is to provide a method of treatment and/or prophylaxis of a disorder such as demyelination disorder, neuro-degenerative disorder, tumor, central neurological disorder, and symptoms associated with myelinogenesis by irradiating light of a specific wavelength such as violet light constantly or at a specific blinking frequency, and an apparatus and a computer program used for the same.

Moreover, an object of the present invention also includes providing a method of treatment and/or prophylaxis of a disorder such as demyelination disorder, neuro-generative disorder, tumor, central neurological disorder and symptoms associated with myelinogenesis in a subject by promoting regeneration or an increase in myelin in at least one area of a central nervous system of the subject, by irradiating light of a specific wavelength to the subject, and an apparatus and a computer program used for the same.

Furthermore, an object of the present invention is also to provide a method for promoting regeneration or an increase in myelin in at least one area of a central nervous system, and a method for promoting survival, proliferation, or differentiation of oligodendrocyte precursor cells or oligodendrocytes in at least one area of the central nervous system.

### Means for Solving the Problems

According to one aspect, the present disclosure is related to a method of treatment and/or prophylaxis of a disorder in a subject requiring treatment and/or prophylaxis, and the method includes irradiating light of a specific wavelength to the subject, and the disorder is selected from a group consisting of demyelination disorder, neuro-degenerative disorder, tumor, central neurological disorder, and symptoms associated with myelinogenesis. The method according to the present disclosure includes treating and/or preventing a disorder in the subject by promoting regeneration or an increase in myelin in at least one area of the central nervous system of the subject by irradiating light of a specific wavelength to the subject, and the disorder is selected from a group consisting of demyelination disorder, neuro-degenerative disorder, tumor, central neurological disorder, and symptoms associated with myelinogenesis.

Moreover, according to one aspect, the present disclosure is related to a method of treatment and/or prophylaxis of a disorder in a subject requiring treatment and/or prophylaxis which includes treating and/or preventing a disorder in the subject by promoting regeneration or an increase in myelin in at least one area of a central nervous of the subject by irradiating light of a specific wavelength to the subject by controlling a light irradiating apparatus, and the disorder is selected from a group consisting of demyelination disorder, neuro-degenerative disorder, tumor, central neurological disorder, and symptoms associated with myelinogenesis. In some embodiments, the light irradiating apparatus includes a light source which irradiates light of a specific wavelength constantly or at a specific blinking frequency to a living body, and a controller which controls the blinking frequency of the light source.

In the context of the present disclosure, the demyelination disorder includes multiple sclerosis, neuromyelitis optica (Devic's syndrome), concentric sclerosis (Balo disease), acute disseminated encephalomyelitis (ADEM), inflammatory diffuse sclerosis (Schilder disease), subacute sclerosing panencephalitis (SSPE), progressive multifocal leukoencephalopathy (PML), hypoxic encephalopathy, central pontine myelinolysis, vitamin B12 deficiency, Guillain-Barre syndrome, and Binswanger disease, but is not restricted thereto.

In the context of the present disclosure, the neuro-generative disorder includes amyotrophic lateral sclerosis (ALS), Alzheimer disease, and spinal damage, but is not restricted thereto.

In the context of the present disclosure, the tumor includes brain tumor (glioma), but is not restricted thereto.

In the context of the present disclosure, the central neurological disorder includes cerebral infarction, but is not restricted thereto.

In the context of the present disclosure, the symptoms associated with myelinogenesis include pain, numbness, leaking of urine, feeling light in one's head, failing vision, and fatigue, but are not restricted thereto.

According to one aspect, the present disclosure is related to a method for promoting regeneration or an increase in myelin in at least one area of a central nervous system of a subject requiring treatment and/or prophylaxis which includes promoting regeneration or an increase in myelin in at least one area of the central nervous system of the subject by irradiating light of a specific wavelength.

Moreover, according to one aspect, the present disclosure is related to a method for promoting survival, proliferation, or differentiation of oligodendrocyte precursor cells or oligodendrocytes in at least one area of a central nervous system of a subject requiring treatment and/or prophylaxis, which includes promoting survival, proliferation, and differentiation of oligodendrocyte precursor cells or oligodendrocytes in at least one area of the central nervous system of the subject.

At the time of application of the method according to the present disclosure, the subject may be a patient who is being administered with or was administered with at least one medication selected from a group consisting of steroids, glatiramer acetate, fingolimod hydrochloride, Siponimod fumaric acid, dimethyl fumarate, interferon β (interferon β-1a or interferon β-1b), natalizumab, and ofatumumab.

The method according to the present disclosure may be applied to a patient for increasing an expression of at least one gene selected from a group consisting of Sox10, Cnp, Mag, Mbp, Mobp, Myrf, Pou3f1, and Trf in at least one area of the central nervous system.

In some embodiments, the light used is violet light.

In some embodiments, the specific wavelength used includes a range of 350 nm to 400 nm, and particularly includes a wavelength of approximately 380 nm.

In some embodiments, the blinking frequency used is either 0 Hz or in a range of 30 Hz to 70 Hz, and in particular, is either 0 Hz or in a range of 35 Hz to 60 Hz, and more specifically, is either 0 Hz or approximately 40 Hz.

In some embodiments, irradiation conditions further include an irradiation time of the light source.

In some embodiments, the light source may be spectacles with a light source or a spectacle frame with a light source, a desktop light source, a mobile terminal mounted light source, a light source installed nearby or in front of face, a portable light source, an indoor lighting, or a desk lamp.

In some embodiments, the subject to which the method according to the present disclosure is applied is a human.

According to one aspect, the present disclosure is related to an apparatus for treatment and/or prophylaxis of a disorder by light stimulation which includes at least one light source which emits light and a driving circuit which drives the light source, and the light emitted by the light source is light of a specific wavelength which produces an effect of treating and/or preventing a disorder by being irradiated to a living body, and the disorder is selected from a group consisting of demyelination disorder, neuro-degenerative disorder, tumor, central neurological disorder, and symptoms associated with myelinogenesis.

Moreover, according to one aspect, the present disclosure is related to an apparatus for promoting regeneration or an increase in myelin by light stimulation which includes at least one light source which emits light, and a driving circuit which drives the light source, and the light emitted from the light source is light of a specific wavelength which produces an effect of promoting regeneration or an increase in myelin by being irradiated to a living body.

According to another aspect, the present disclosure is related to an apparatus which accelerates survival, proliferation, or differentiation of oligodendrocyte precursor cells or oligodendrocytes by light stimulation, which includes at least one light source which emits light, and a driving circuit which drives the light source, and the light emitted from the light source is light of a specific wavelength which produces an effect of promoting survival, proliferation, or differentiation of oligodendrocyte precursor cells or oligodendrocytes by being irradiated to a living body.

In some embodiments, the driving circuit in the apparatus according to the present disclosure includes at least one processor which is communicably connected to the light source and at least one memory, and at least one memory for storing commands that are executable by the processor.

According to one aspect, the present disclosure is related to a method of operating an apparatus according to the present disclosure including a light source which irradiates light of a specific wavelength constantly or at a specific blinking frequency to a living body and a controller which controls the blinking frequency of the light source, which includes controlling the blinking frequency of the light source either to 0 Hz or in a range of 30 Hz to 75 Hz by the controller, and irradiating light of a wavelength in a range of 350 nm to 400 nm.

According to one aspect, the present disclosure is related to a computer program which causes an apparatus including a light source which irradiates light of a specific wavelength constantly or at a specific blinking frequency to a living body and a controller which controls the blinking frequency of the light source, to execute a method of operating according to the present disclosure.

According to the method according to the present disclosure, the survival, proliferation, and differentiation of oligodendrocyte precursor cells or oligodendrocytes of a living body having received light are accelerated, and it is possible to accelerate regeneration or an increase in myelin. Promoting the regeneration or an increase in myelin leads to treatment and/or prophylaxis of a disorder such as demyelination disorder, neuro-degenerative disorder, tumor, central neurological disorder, and symptoms associated with myelinogenesis.

In the method and apparatus according to the present disclosure, the light is violet light for example. According to this invention, since it is possible to irradiate violet light of a wavelength outside the visible light region to a living body, it is possible to have an effect on the living body without feeling flickering or dazzling as in a case of white light. Moreover, the violet light is light of a wavelength in a range of 360 nm to 400 nm, and light of this wavelength has a low visible sensitivity as compared to that of white light, and is light of a wavelength region which imparts no uncomfortable feeling or hardly any uncomfortable feeling to a living body (particularly human).

In some embodiments of the present disclosure, light of a wavelength in a range of 350 nm to 400 nm, such as light of one of wavelengths 350 nm, 360 nm, 370 nm, 380 nm, 390 nm, or 400 nm, or light of an arbitrary wavelength included in a range specified by the abovementioned arbitrary wavelength (for example, in a range of 370 nm to 390 nm) may be used. In some embodiments of the present disclosure, a wavelength of approximately 380 nm is included. Moreover, the term 'approximately' used in the present specification indicates that it includes any value that is within a variation of up to ±5% of the value modified by this term.

In the method and apparatus according to the present disclosure, the condition for irradiation of the light is that the light is lit constantly (in other words, 0 Hz) or irradiated at a blinking frequency in a range of higher than 0 Hz up to 150 Hz.

In some embodiments of the present disclosure, for the light which is irradiated constantly (0 Hz) or the light which is irradiated at a blinking frequency in the range of 30 Hz to 75 Hz, for instance, light blinking at any of the blinking frequencies 30 Hz, 35 Hz, 40 Hz, 45 Hz, 50 Hz, 55 Hz, 60 Hz, 65 Hz, 70 Hz, or 75 Hz, or light of a an arbitrary blinking frequency included in a range specified by any of the abovementioned arbitrary blinking frequencies (for example, in a range of 35 Hz to 45 Hz) may be used. In some embodiments of the present disclosure, the blinking frequency is approximately 40 Hz.

In the method and apparatus according to the present disclosure, the light can be irradiated in a range of irradiance of 0.5 µW/cm² to 1000 µW/cm² for example, or in a range of irradiance of 0.1 µW/cm² (0.001 W/m²) to 5000 µW/cm² (50 W/m²). According to some embodiments, by irradiating light such as violet light in the abovementioned range of irradiance, it is possible to have an effect on oligodendrocytes. Even when it is a weak light of an extremely small amount (light with a weak optical sensitivity) in particular, it has been verified that a characteristic phenomenon may occur.

In the method and apparatus according to the present disclosure, the controller of the apparatus, by transceiving to and from an isolated controller such as a portable terminal, is capable of carrying out control by changing irradiation conditions of light (including irradiating constantly or at a blinking frequency) such as irradiance, irradiation time, irradiation-start time, irradiation-end time, and irradiating constantly or at a blinking frequency. According to some embodiments, the abovementioned irradiation conditions being subjected to isolated control, it is possible to set arbitrarily irradiation conditions appropriate for inducing control of oligodendrocytes, and have the desired effect.

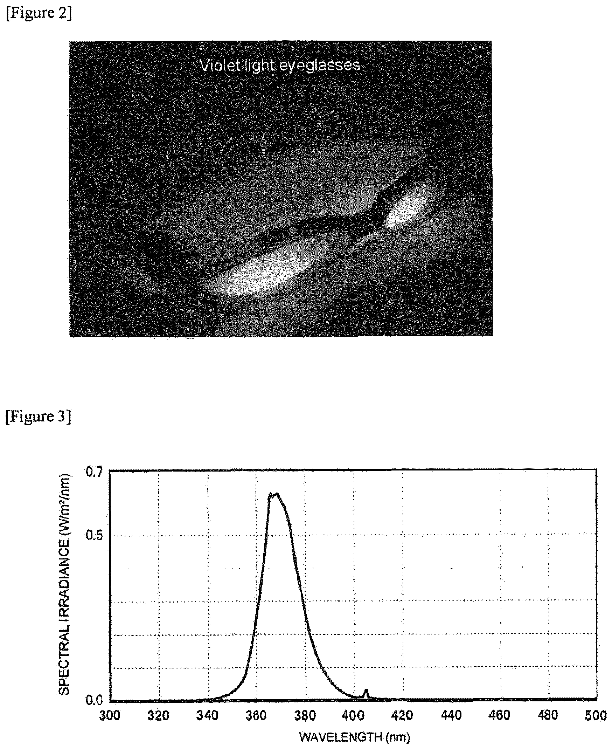

In the method and apparatus according to the present disclosure, the light source may be a light source installed nearby or in front of face, such as spectacles with a light source (for example, refer to Fig. 2) or a spectacle frame with a light source, a desktop light source, and a mobile terminal mounted light source. According to some embodiments, as it is possible to irradiate specific light from the light source installed nearby or in front of face such as spectacles with a light source or spectacle frame with a light source that can be worn easily and have no uncomfortable feeling in daily use, they are highly practical, and are capable are irradiating light any time even in various situations and varied environments.

In the method and apparatus according to the present disclosure, the light source may be a non-mounting type light source such as a portable light source, or may be a mounting type light source such as an indoor lighting, a table stand, and a dedicated device. According to some embodiments, it is possible to make it an apparatus in various forms of light source appropriate for an environment of usage. For instance, the light source may be used in combination with a glass, a spectacle lens, or a contact lens that allows violet light to pass through. Moreover, sunlight that has passed through a glass, a spectacle lens, or a contact lens that allows violet light to pass through, may be used as the light source.

The method according to the present disclosure is a method of controlling oligodendrocytes by irradiating light of a specific wavelength constantly or at a specific blinking frequency to a living body (such as a mammal including a human) which is characterized by controlling an emission of light having controlled the gene expression of oligodendrocytes of the living body which has received the light.

The apparatus according to the present disclosure is an apparatus for treating or preventing a disorder by irradiating violet light constantly to a living body, which is characterized by comprising a light source which emits the violet light, and a light-emission time controller which irradiates the violet light for a specific time or a specific period of time, and the disorder is selected from a group consisting of demyelination disorder, neuro-degenerative disorder, tumor, central neurological disorder, and symptoms associated with myelinogenesis.

Furthermore, as it is described below while referring to the accompanying diagrams, in some embodiments of the present disclosure, a method of treatment and/or prophylaxis of a disorder in a subject requiring treatment and/or prophylaxis, a method for promoting regeneration or an increase in myelin in at least one area of the central nervous system of the subject requiring treatment and/or prophylaxis, a method for promoting survival, proliferation, or differentiation of oligodendrocyte precursor cells or oligodendrocytes in at least one area of the central nervous system, an apparatus for treating and/or preventing a disorder by light stimulation, an apparatus for promoting regeneration or increase in myelin by light stimulation, an apparatus for promoting survival, proliferation, or differentiation of oligodendrocyte precursor cells or oligodendrocytes by light stimulation, a method of operating the apparatus, and a computer program which causes to execute the method of operating, are provided.

### Brief Description of the Drawings

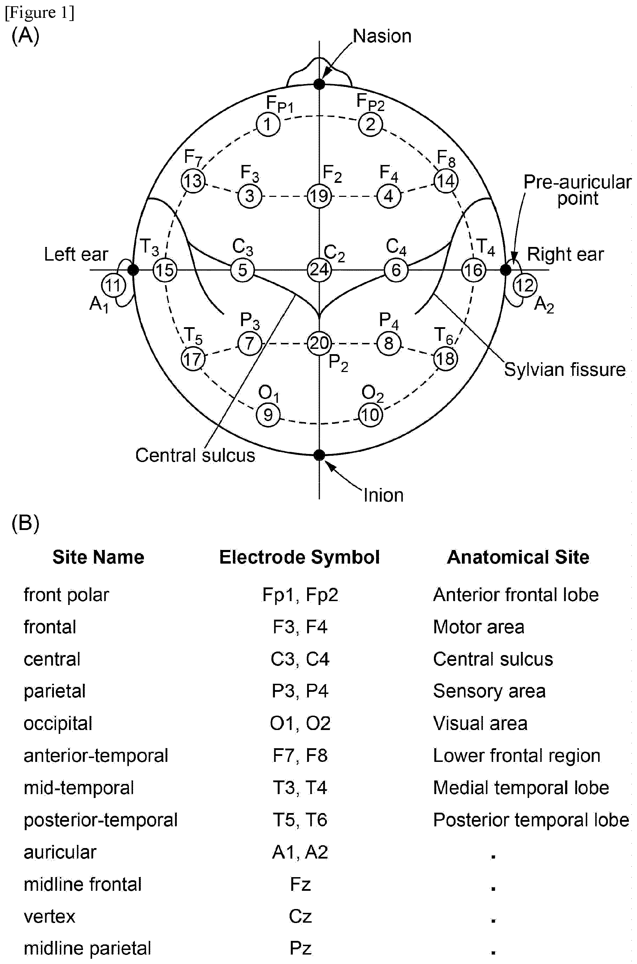

Fig. 1 is an illustration diagram of various sites of a brain;
Fig. 2 is an example of violet light spectacles which irradiate violet light;
Fig. 3 is a graph showing a relationship of wavelength and spectral irradiance of violet fluorescent light;
Fig. 4 is a light spectrum of an LED with a peak wavelength of 375 nm;
Fig. 5 is a block diagram of an embodiment of an apparatus for controlling biological function according to the present invention;
Fig. 6 is a boxplot in which genes associated with myelinogenesis and oligodendrocyte differentiation out of genes for which an expression is up-regulated by violet light (VL) are shown, where WL denotes white light and VL denotes violet light;
Fig. 7 is a diagram in which an expression pattern of an oligodendrocyte differentiation marker molecule is shown, where, OPC denotes oligodendrocyte precursor cell, pre-OL denotes pre-oligodendrocyte, and OL denotes matured oligodendrocyte;
Fig. 8 is a diagram of photographs showing MBP and DNA in cerebral cortex acquired from mice of an experimental group (VL) and mice of a control group (WL);
Fig. 9 is a diagram showing sites of a cerebral cortex acquired from a mouse of the experimental group (VL) and a mouse of the control group (WL);
Fig. 10 is a graph in which the mice of the experimental group (VL) and the mice of the control group (WL) are compared in an area in which MBP is expressed;
Fig. 11 shows graphs in which gene expression in a tissue of hippocampus of brain for SOX10, CNP, MAG, MBP, Mobp, and Myrf which are biomarkers of oligodendrocyte is verified by quantitative PCR;
Fig. 12 shows photographs indicating expression of c-Fos, MBP, CC1, and Iba1 in a nucleus accumbens of brain;
Fig. 13 is a diagram showing a site of the nucleus accumbens of brain;
Fig. 14 show graphs comparing a depression-induced experimental group (cVL) with a depression-induced control group (WL) and a control group with no depression induced (WL) for the expression of c-Fos, MBP, CC1, and Iba1 in the nucleus accumbens of brain;
Fig. 15 show photographs indicating expression of c-Fos, MBP, CC1, and Iba1 in a prefrontal cortex of brain;
Fig. 16 is a diagram showing a site of the prefrontal cortex in brain;
Fig. 17 show graphs comparing a depression-induced experimental group (cVL) with a depression-induced control group (WL) and a control group with no depression induced (WL) for the expression of c-Fos, MBP, CC1, and Iba1 in the prefrontal cortex of brain;
Fig. 18 is a graph comparing a depression-induced experimental group (cVL) with a depression-induced control group (WL) and a control group with no depression induced (WL) for the expression of FGF1 in the prefrontal cortex;
Fig. 19 is a graph comparing a depression-induced experimental group (cVL) with a depression-induced control group (WL) and a control group with no depression induced (WL) for the expression of FGF22 in the prefrontal cortex of brain;
Fig. 20 is a graph comparing an experimental group (VL) with a control group (WL) for the expression of FGF21 and mRNA in the hippocampus of a left brain and a right brain, where, 'left' indicates the left brain and 'right' indicates the right brain; and
Fig. 21 is a graph comparing the expression of MAG (40 Hz VL) in an animal model for Alzheimer disease of a P301S mutant human tau gene injected mouse (PS19) to which violet light of 40 Hz was irradiated, with a control group (cWL).

### Mode for Carrying Out the Invention

A method of treatment and/or prophylaxis of a disorder in a subject requiring treatment and/or prophylaxis, a method for promoting regeneration or an increase in myelin in at least one area of a central nervous system of the subject requiring treatment and/or prophylaxis, a method for promoting survival, proliferation, or differentiation of oligodendrocyte precursor cells or oligodendrocytes in at least one area of the central nervous system, an apparatus for treatment and/or prophylaxis of a disorder by light stimulation, an apparatus for promoting regeneration or an increase in myelin by light stimulation, an apparatus for promoting survival, proliferation, or differentiation of oligodendrocyte precursor cells or oligodendrocytes by light stimulation, a method of operating the apparatus, and a computer program which causes to execute the method of operating according to the present disclosure will be described below while referring to the accompanying diagrams. However, the present invention is not restricted to content of embodiments and examples described below, and includes various modified examples and application examples within the scope of the present invention.

### [Method of treatment and/or prophylaxis of disorder]

The method of treatment and/or prophylaxis of a disorder according to the present disclosure is characterized by including irradiating light of a specific wavelength to a subject requiring treatment and/or prophylaxis.

The inventors of the present invention discovered that by irradiating the light of a specific wavelength to the subject, it is possible to accelerate survival, proliferation, and differentiation of oligodendrocyte precursor cells or oligodendrocytes in at least one area of the central nervous system of the subject and to activate nerves, thereby activating immunity. Therefore, one aspect of the present disclosure is related to a method for promoting survival, growth or differentiation of oligodendrocyte precursor cells or oligodendrocytes in at least one area of the central nervous system in the subject requiring treatment and/or prophylaxis, and this method includes promoting survival, proliferation, or differentiation of oligodendrocyte precursor cells or oligodendrocytes in at least one area of the central nervous system of the subject by irradiating light of a specific wavelength to the subject. Here, the subject is a living body having oligodendrocytes, and may be a mammal, particularly a human.

Oligodendrocytes are one of glial cells in the central nervous system, and are also called as oligodendroglial cells. Among cells that form the central nervous system, there are nerve cells that exchange information by transmitting electric signals, and glial cells (neurogliacytes) that supply nutrition to nerve cells, thereby helping a function thereof. Oligodendrocytes that are classified as glial cells, in particular, have a function of myelinogenesis (formation of myelin sheath) which covers an axon portion of a nerve cell. The oligodendrocytes, upon undergoing a series of steps such as proliferation, migration, differentiation, and myelination, eventually have their axons myelinated. Mielin functions as an insulator, accelerates the speed of transmitting electric signal, and prevents mixing of information transmitted by other axons due to leaking of an electric signal to an outside of the axon. The demyelination disorder is an intractable disease of unknown etiology in which myelin which covers nerves disappears (demyelination), and nerve signals cannot be transmitted properly, thereby causing various neurological symptoms such as numbness of four limbs. Suppressing demyelination and inducing remyelination are considered to be a key to the treatment of the demyelination disorder. Oligodendrocytes have been known to be involved in various disorders, and for instance, it has been known that the oligodendrocytes decrease with aging, it has been known that oligodendrocytes decrease in Alzheimer disease, it has been known that differentiation of oligodendrocytes is significant for recovery from cerebral infarction, and it has been known that differentiation of oligodendrocytes is significant for recovery from spinal damage.

The acceleration of the survival, proliferation, and differentiation of oligodendrocyte precursor cells or oligodendrocytes is indicated by an increase in expression of at least one gene selected from a group consisting of Sox10, Cnp, Mag, Mbp, Mobp, Myrf, Pou3f1, and Trf in at least one area of the central nervous system. The `at least one area' of the central nervous system may be, for instance, rhinencephalon, amygdaloid nucleus, corpus striatum, hippocampus, cerebral neocortex, epithalamus, optic thalamus, hypothalamus, subthalamus, pituitary gland, pineal corpus, third cerebroventricle, lamina tectoria, cerebral peduncle, pretectum, cerebral aqueduct, pontine, cerebellum, medulla oblongata, and spinal cord, but is not restricted thereto. The inventors of the present invention discovered that, by irradiating light of a specific wavelength to a subject, the expression of Sox10, Cnp, Mag, Mbp, Mobp, Myrf, Pou3f1, and Trf in the central nervous system of the subject, and particularly in the hippocampus, increases. Therefore, one aspect of the present disclosure is related to a method of increasing the expression of at least one gene selected from a group consisting of Sox10, Cnp, Mag, Mbp, Mobp, Myrf, Pou3f1, and Trf in at least one area of the central nervous system of the subject. The acceleration of the survival, proliferation, and differentiation of oligodendrocyte precursor cells and oligodendrocytes leads to the acceleration of regeneration or an increase in myelin, consequently leading to the treatment and/or prophylaxis of a disorder such as demyelination disorder, neuro-degenerative disorder, tumor, central neurological disorder, and symptoms associated with myelinogenesis. Therefore, one aspect of the present disclosure is related to a method for promoting regeneration or an increase in myelin in at least one area of the central nervous system of the subject requiring treatment and/or prophylaxis, and the method includes promoting the regeneration or an increase in myelin in at least one area of the central nervous system of the subject by irradiating light of a specific wavelength to the subject. Here, the subject may be a living body having myelin, such as a mammal, particularly a human.

The regeneration or an increase in myelin of the subject can be evaluated by visualizing myelin by MRI by using a method called as myelin mapping (for example, refer to Fujiyoshi et al., J Neurosci. 2016 Mar 2;36(9); 2796-808.). In the myelin mapping, it is possible to photograph myelin in approximately 10 minutes by using MRI (3 Tesla (3T) apparatus), and to visualize remyelination easily.

As mentioned above, one aspect of the present disclosure is related to the method of treatment and/or prophylaxis of a disorder in a subject requiring treatment and/or prophylaxis which includes irradiating light of a specific wavelength to the subject, and the disorder is selected from a group consisting of demyelination disorder, neuro-degenerative disorder, tumor, central neurological disorder, and symptoms associated with myelinogenesis. Moreover, the method may include treating and/or preventing a disorder in a subject by promoting regeneration or an increase in myelin in at least one area of the central nervous system of the subject by irradiating light of a specific wavelength to the subject.

The demyelination disorder may be any one of multiple sclerosis, neuromyelitis optica (Devic's syndrome), concentric sclerosis (Balo disease), acute disseminated encephalomyelitis (ADEM), inflammatory diffuse sclerosis (Schilder disease), subacute sclerosing panencephalitis (SSPE), progressive multifocal leukoencephalopathy (PML), hypoxic encephalopathy, central pontine myelinolysis, vitamin B12 deficiency, Guillain-Barre syndrome, and Binswanger disease.

Multiple sclerosis is one of the demyelination disorders of the central nervous system, and is a chronic inflammatory disorder characterized by a damage of myelin and a dysfunction of brain, nervous system, and spinal cord. The exact cause of development of the multiple sclerosis is not known. When the multiple sclerosis is developed, subject's own immunity system attacks the myelin sheath, and 'demyelination', in which, an axon is laid bare, occurs. When the demyelination occurs, nerve conduction is not carried out appropriately, and neurological symptoms start appearing. The main symptoms are, symptoms such as visual field disorder, multiple vision disorder, disorder of sensation, motility disorder, gait disorder, urination disorder, articulation disorder, and higher cerebral dysfunction. The multiple sclerosis (MS) can be broadly classified into four types, that are, relapsing and remitting multiple sclerosis (RRMS), secondary progressive multiple sclerosis (SPMS), primary progressive multiple sclerosis (PPMS), and progressive relapsing multiple sclerosis (PRMS).

For the treatment of multiple sclerosis, methylprednisolone large-dose drip infusion pulse therapy and plasma exchange therapy are used often as an acute short-term therapy. While short term effects of these therapies have been acknowledged, the multiple sclerosis being a disorder in which the disease activity persists chronically, a therapeutic method aimed at prognostic improvement over a long term has been sought. As a therapeutic medication, glatiramer acetate, fingolimod hydrochloride, Siponimod fumaric acid, dimethyl fumarate, interferon β, natalizumab, and ofatumumab can be used in addition to steroids such as methylprednisolone. Therefore, in some embodiments of methods according to the present disclosure, the subject may be a patient which is being administered with or was administered with at least one medication selected from a group consisting of steroids, glatiramer acetate, fingolimod hydrochloride, Siponimod fumaric acid, dimethyl fumarate, interferon β, natalizumab, and ofatumumab. By a therapy in which administration of these medications and the light stimulation according to the present invention are combined, the disorder may be controlled more effectively. Therefore, one aspect of the present disclosure is related also to a method of treatment and/or prophylaxis of demyelination disorder in the subject requiring treatment and/or prophylaxis which includes irradiating light of a specific wavelength to the subject, and administering at least one medication selected from a group consisting of steroids, glatiramer acetate, fingolimod hydrochloride, Siponimod fumaric acid, dimethyl fumarate, interferon β, natalizumab, and ofatumumab to the subject.

The neuro-degenerative disorder, for instance, may be any one of amyotrophic lateral sclerosis (ALS), Alzheimer disease, and spinal damage, but is not restricted thereto.

The tumor, for instance, may be brain tumor (glioma), but is not restricted thereto.

The central neurological disorder, for instance, may be cerebral infarction, but is not restricted thereto.

The symptoms associated with myelinogenesis, for instance, may be pain, numbness, leaking of urine, feeling light in one's head, failing vision, and fatigue, but are not restricted thereto.

In some embodiments of the method according to the present disclosure, the light used may be violet light. Moreover, in some embodiments of the method according to the present disclosure, the specific wavelength used may include a wavelength in a range of 350 nm to 400 nm, and may be approximately 380 nm specifically. The blinking frequency, for example, may be 0 Hz or in a range of 30 Hz to 70 Hz, and 0 Hz or in a range of 35 Hz to 60 Hz specifically. The blinking frequency, may be 0 Hz or approximately 40 Hz specifically. The irradiation conditions may further include an irradiation time of the light source. The light source may be spectacles with a light source or a spectacle frame with a light source, a desktop light, a mobile terminal mounted light source, a light source installed nearby or in front of face, a portable light source, an indoor lighting, and a desk lamp, but is not restricted thereto.

In some embodiments of the present disclosure, the specific time of irradiating light may be time in an arbitrary range of 10 seconds to 24 hours per day, and may be any one of 10 seconds, 30 seconds, 45 seconds, one minute, three minutes, five minutes, 10 minutes, 15 minutes, 30 minutes, 45 minutes, one hour, two hours, three hours, four hours, five hours, six hours, seven hours, eight hours, nine hours, 10 hours, 11 hours, 12 hours, 18 hours, and 24 hours, or may be an arbitrary time included in a range (for example, a range of one hour to 12 hours) stipulated by the abovementioned arbitrary time. The specific period for which the light is irradiated may be, for example, one day, two days, three days, four days, five days, six days, one week, two weeks, three weeks, one month, two months, three months, six months, one year, two years, three years, or a period longer than these periods.

One aspect of the present disclosure is related to a method of treatment and/or prophylaxis of a disorder in a subject requiring treatment and/or prophylaxis which includes irradiating light of a specific wavelength by controlling a light irradiating apparatus, thereby treating and/or preventing a disorder in the subject by promoting regeneration or an increase in myelin in at least one area of a central nervous system of the subject, and the disorder is selected from a group consisting of demyelination disorder, neuro-degenerative disorder, tumor, central neurological disorder, and symptoms associated with myelinogenesis. In some embodiments, the light irradiating apparatus is capable of irradiating violet light. Moreover, in some embodiments, the light irradiating apparatus is capable of irradiating light of a wavelength in a range of 350 nm to 400 nm, and specifically, light of a wavelength of approximately 380 nm. The blinking frequency of the light irradiating apparatus may be controllable to 0 Hz, or in a range of 30 Hz to 70 Hz for example, and specifically, to 0 Hz or in a range of 35 Hz to 60 Hz, the blinking frequency, specifically, may be 0 Hz or approximately 40 Hz. The irradiation time of the light irradiating apparatus may be controllable. The light source may be in the form of spectacles with a light source or a spectacle frame with a light source, a desktop light source, a mobile terminal mounted light source, a light source installed nearby or in front of face, a portable light source, an indoor lighting, and a desk lamp, but is not restricted thereto.

### [Apparatus]

One aspect of the present disclosure is related to an apparatus for treatment and/or prophylaxis of a disorder by light stimulation. In some embodiments, the apparatus for treatment and/or prophylaxis of a disorder according to the present disclosure may include at least one light source which emits light, and a driving circuit which drives the light source. Here, the light emitted by the light source is a light of specific wavelength which produces an effect of treating and/or preventing a disorder by being irradiated to a living body.

Moreover, one aspect of the present disclosure is related to an apparatus for promoting regeneration or an increase in myelin by light stimulation. In some embodiments, the apparatus for promoting regeneration or an increase in myelin according to the present disclosure may include at least one light source which emits light and a driving circuit which drives the light source. Here, the light emitted by the light source is light of a specific wavelength which produces an effect of promoting regeneration or an increase in myelin by being irradiated to a living body.

Furthermore, one aspect of the present disclosure is related to an apparatus which accelerates survival, proliferation, or differentiation of oligodendrocyte precursor cells or oligodendrocytes by light stimulation. In some embodiments, the apparatus according to the present disclosure which accelerates survival, proliferation, or differentiation of oligodendrocyte precursor cells or oligodendrocytes may include at least one light source which emits light, and a driving circuit which drives the light source. Here, the light emitted by the light source is light of a specific wavelength which produces an effect of promoting survival, proliferation, or differentiation of oligodendrocyte precursor cells or oligodendrocytes by being irradiated to a living body.

In the present specification, the apparatus for treatment and/or prophylaxis of a disorder by light stimulation, the apparatus for promoting regeneration or an increase in myelin by light stimulation, and the apparatus for promoting survival, proliferation, or differentiation of oligodendrocyte precursor cells or oligodendrocytes, in some cases, are collectively referred to as 'apparatus for controlling biological function', or just 'apparatus'.

These apparatuses, by controlling the irradiation conditions of light, are capable of stimulating oligodendrocyte precursor cells or oligodendrocytes of a living body, and activating nerves and activating immunity. As described heretofore, a specific gene expression is enhanced in the oligodendrocyte precursor cells or oligodendrocytes subjected to light stimulation, and survival, proliferation, and differentiation are accelerated, and as a result, the regeneration or an increase in myelin is accelerated, and further as a result thereof, leading to treatment and/or prophylaxis of the disorder.

In some embodiments, the apparatus according to the present disclosure, as mentioned above, is an apparatus for treatment and/or prophylaxis of a disorder by controlling biological functions such as survival, proliferation, or differentiation of oligodendrocyte precursor cells or oligodendrocytes, and myelinogenesis by irradiating violet light constantly or at a specific blinking frequency to a living body, and activating nerves and activating immunity, is characterized by including a light source which emits the violet light, a light-emission cycle controller which makes irradiate the violet light constantly or at a specific blinking frequency, and a light-emission time controller which makes irradiate the violet light for a specific time or for a specific period of time, and by being an apparatus used for treatment and/or prophylaxis of a disorder which controls the biological function, and the disorder is selected from a group consisting of demyelination disorder, neuro-degenerative disorder, tumor, central neurological disorder, and symptoms associated with myelinogenesis.

Moreover, the apparatus according to the present disclosure, in some embodiments, is an apparatus which controls biological function by irradiating violet light constantly to a living body, and is characterized by including a light source which emits the violet light, and a light-emission time controller which makes irradiate the violet light for a specific time or a specific period of time.

Moreover, the apparatus according to the present disclosure, in some embodiments, is related to an apparatus used for treatment and/or prophylaxis of the disorder which includes at least one light source which emits light, and a driving circuit which drives the light source, and the light controls a biological function by light stimulation by light of a specific wavelength which produces an effect by being irradiated to a living body. Therefore, one aspect of the present disclosure is related to an apparatus for treatment and/or prophylaxis of a disorder by light stimulation which is configured to include at least one light source which emits light and a driving circuit which drives the light source, and the driving circuit includes at least one processor which is communicably connected to the light source and at least one memory, and at least one memory for storing commands that are executable by the processor, and the light source emits light of a specific wavelength which produces an effect of treating and/or preventing a disorder by being irradiated to a living body. Moreover, one aspect of the present disclosure is related to an apparatus for promoting regeneration or an increase in myelin by light stimulation which is configured to include at least one light source which emits light and a driving circuit which drives the light source, and the driving circuit includes at least one processor which is communicably connected to the light source and at least one memory and at least one memory for storing commands that are executable by the processor, and the light source emits light of a specific wavelength which produces an effect of promoting regeneration or an increase in myelin by being irradiated to a living body. Furthermore, one aspect of the present disclosure is related to an apparatus for promoting survival, proliferation, or differentiation of oligodendrocyte precursor cells or oligodendrocytes by light stimulation which is configured to include at least one light source which emits light and a driving circuit which drives the light source, and the driving circuit includes at least one processor which is communicably connected to the light source and at least one memory, and at least one memory for storing commands that are executable by the processor, and the light source emits light of a specific wavelength which produces an effect of promoting survival, proliferation, and differentiation of oligodendrocyte precursor cells or oligodendrocytes by being irradiated to a living body.

### (Light source)

The wavelength of light emitted by the light source is not restricted, and in some embodiments, violet light defined by a wavelength range of 360 nm to 400 nm is used.

A light source with a possible oscillating frequency in a range of 0 Hz (constantly irradiated light, direct light) to 150 Hz can be used preferably. The frequency can be adjusted in units of 0.5 Hz and 1 Hz by setting of the controller, and light of an arbitrary blinking frequency can be produced. As the blinking frequency is increased, there is an advantage that the blinking is not sensed any more, although this perception varies from person to person. The blinking frequency is not restricted to 10 Hz and 60 Hz used in experimental examples.

The irradiance from the light source may be variable or may be a constant value. Although irradiance with maximum output of 310 µW/cm² is used in some embodiments, it is not restricted to this value. The light source may be configured arbitrarily for irradiance in a range of 0.1 µW/cm² (0.001 W/m²) to 5000 µW/cm² (50 W/m²) for example, or irradiance in a range of 1 µW/cm² (0.01 W/m²) to 1000 µW/cm² (10 W/m²) for example, or irradiance in a range of 0.5 µW/cm² (0.005 W/m²) to 500 µW/cm² (5 W/m²) for example, or irradiance in a range of 0.5 µW/cm² to 1000 µW/cm². Furthermore, since a light source with such irradiance can be used easily for spectacles or a spectacle frame, and other portable irradiation equipment, it can be worn even in a day-to-day life. Even with weak light of extremely small amount (light with weak optical sensitivity) in particular, an occurrence of a characteristic phenomenon has been verified, and an effect on various parts of a living body including brain, and an application in cell activity (used with a significance including gene expression control) can be anticipated.

Light may be specified in terms of relative luminosity. Since characteristics of the present invention can be realized even with a low relative luminosity, the violet light which generates stimulation in a living body can be irradiated while blinking, at a low relative luminosity, and a desired site can be stimulated without strain on a living body.

It is preferable to set the irradiation time of light arbitrarily according to the purpose thereof, and it may be a short time or a long time. The light can be irradiated intermittently (at regular interval or irregular interval) or continuously. The time of irradiation of light can be set to be a period of time between 8 am to 1 pm, between 9 am to 12 pm, between 10 am to 11 am, and the period can be set to be at least 10 minutes, at least 15 minutes, at least 20 minutes, at least 30 minutes, at least one hour, at least two hours, at least three hours, at least four hours, or at least five hours. In some embodiments, irradiation for a period of three hours from 9 am to 12 pm, or for a period of two hours from 9 am to 11 am is used. In a case of irradiation in which the time has been set in this manner, a timer function can be used.

The light source may be spectacles or a spectacle frame with a light source. Such spectacles or spectacle frame being easy to wear, and having a light source which emits light at a blinking frequency fitted to spectacles or spectacle frame with no uncomfortable feeling on a daily basis, they are highly practical, and can be worn any time even in various situations and varied environments. Moreover, the light source may be a desktop light source, or a light source installed nearby or in front of face such as a mobile terminal mounted light source, or a non-mounting type light source such as a portable light source, or may be a mounting type light source such as an indoor lighting, a table stand, and a dedicated device, and can be an apparatus in various forms of light source appropriate for an environment of usage.

### (Controller)

The controller is a section which controls irradiation conditions (irradiate constantly or at a blinking frequency) of light from the light source. The controller may be provided with an electric power source for supplying electric power to the light source, and such electric power source may be a battery, or may have a wire drawn to a battery installed at a different location. Moreover, in a case of being immovable at one location, it may be connected to a household electric power supply.

It is preferable that the controller, by tranceiving to and from an isolated controller such as a portable terminal, changes irradiation conditions of light such as blinking frequency, irradiance, irradiation time, irradiation-start time, and irradiation-end time. Since such controller carries out an isolated control of various irradiation conditions mentioned above, it is possible to have the desired effect by setting arbitrarily the irradiation conditions appropriate for controlling the desired biological function.

Furthermore, the controller may have controller functions and timer functions of the light source. The controller functions include functions such as changing the frequency and irradiance, and setting the irradiation time. Moreover, the timer functions include an ability to set the irradiance time of light. Such controller functions and time functions may have been provided integrally with the instrument, or may be provided as separate members.

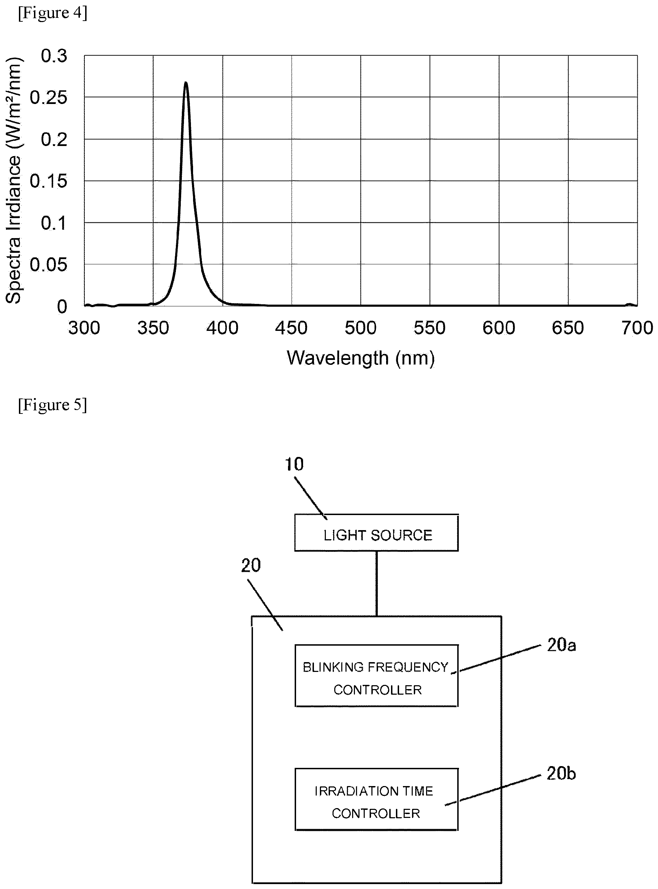

A block diagram of a simplified example of an apparatus which can be used for the abovementioned control of biological functions as an embodiment of the apparatus according to the present disclosure is shown in Fig. 5. The apparatus shown in Fig. 5 may include various functions of the apparatus for treatment and/or prophylaxis of a disorder by light stimulation, the apparatus for promoting regeneration or an increase in myelin by light stimulation, and the apparatus for promoting survival, proliferation, and differentiation of oligodendrocyte precursor cells and oligodendrocytes by light stimulation (these apparatuses can be collectively referred to as 'apparatus for controlling biological function') described in the present specification, and therefore, all the abovementioned apparatuses in the present specification can be represented by the block diagram in Fig. 5. The apparatus for controlling biological function can have a light source 10 and a controller 20. The light source 10 irradiates light of a specific wavelength. It is preferable that a wavelength of light irradiated by the light source 10 includes VL (violet light) or a wavelength range of 350 nm to 400 nm described heretofore, and more preferably, includes a wavelength of 380 nm. The light source 10 may be an arbitrary light source, and a light emitting diode (LED) can be used preferably from viewpoints such as small size, long life, and ease of blinking control (refer to Fig. 3 for an example of a spectrum of violet fluorescent light and Fig. 4 for an example of a spectrum of an LED). The number of light sources 10 may be one or may be plural depending on factors such as an intended intensity of irradiation and an intended range of irradiation of the light source.

The controller 20 is connected to the light source 10 by a wired connection or a wireless connection, and is configured to control irradiation conditions of the light source 10. The irradiation conditions can include at least one of the blinking frequency and the irradiation time of the light source 10, and therefore, the controller 20 can include at least one of a blinking frequency controller 20a and an irradiation time controller 20b. The blinking frequency may be preferably 0 Hz or in a range of 30 Hz to 75 Hz, and more preferably 0 Hz or in a range of 35 Hz to 45 Hz, and even more preferably 0 Hz or 40 Hz specifically. The blinking frequency of 0 Hz refers to constantly irradiated light. The irradiation time can be set arbitrarily to be in a range of 10 seconds to 24 hours per day for example, and a specific time for which the irradiation is to be continued can be set arbitrarily for a period of one day to few years, or more than that for example.

The controller 20 can include a processor such as a CPU (Central Processing Unit), and executes a processing of controlling irradiation conditions of the light source 10. The processing carried out by the controller 20 may be realized by a computer program or may be realized by hardware by a logic circuit. The computer program may be stored in a computer-readable recording medium. The recording medium having the computer program recorded may be a non-transient recording medium. The non-transient recording medium is not restricted in particular, and may be a recording medium such as a memory card and a CD-ROM (Compact Disc - Read Only Memory). The computer program stored in the recording medium can be installed in a computer unit via an appropriate reader. Examples of appropriate reader are a card reader in a case in which the recording medium is a memory card and a CD (Compact Disc) drive in a case in which the recording medium is a CD-ROM. Moreover, computer program may be a computer program downloaded to a computer unit via a communication network from an external server.

In the apparatus according to the present disclosure, the driving circuit may include at least one processor which is communicably connected to the light source and at least one memory, and at least one memory for storing commands that are executable by the processor.

The light source 10 of the apparatus for controlling biological function shown in Fig. 5 may be spectacles with a light source or a spectacle frame with a light source, a desktop light source, a mobile terminal mounted light source, a light source installed nearby or in front of face, a portable light source, an indoor light, or a table stand. Moreover, the apparatus for controlling biological function may be provided as a product with a light source having at least the light source 10 mounted thereon, out of the light source 10 and the controller 20, including spectacles or a spectacle frame, a desktop light, a mobile terminal, a case for a mobile terminal, a head-worn article (such as cap, earphone headphone), a portable light, an indoor light, or a table stand.

As described heretofore, the apparatus for controlling biological function by light stimulation according to the present disclosure can lead to treatment and/or prophylaxis of the disorder, acceleration of regeneration and an increase in myelin, acceleration of survival, proliferation or differentiation of oligodendrocyte precursor cells or oligodendrocytes by irradiating light of a specific wavelength such as violet light constantly or at a specific blinking frequency to a living body.

Moreover, one aspect of the present invention is related to a method of operating an apparatus for controlling biological function by light stimulation. Accordingly, some embodiments of the present disclosure are related to a method of operating the apparatus used for controlling a biological function, and the apparatus includes a light source which irradiates light of a specific wavelength constantly or at a specific blinking frequency to a living body, and a controller which controls the blinking frequency of the light source, and the apparatus irradiates light of a specific wavelength constantly or at a specific blinking frequency to a living body. Here, the blinking frequency of the light source may be controlled to be 0 Hz or to be in a range of 30 Hz to 75 Hz, and moreover, the light source may be a light source which irradiates light of a wavelength in a range of 350 nm to 400 nm to a living body. In addition, one aspect of the present invention is related to a computer program which causes an apparatus including a light source which irradiates light of a specific wavelength constantly or at a specific blinking frequency to a living body, and a controller which controls a blinking frequency of the light source, to execute the method of operating.

The computer program according to the present disclosure may have a command stored in a non-transitory computer-readable medium. The computer program according to the present disclosure can execute predetermined steps when the command is executed by the processor. Accordingly, one aspect of the present disclosure is related also to a computer-readable medium which is a non-transitory computer-readable medium having commands stored therein, and as the command is executed by a processor, it is capable of executing for an apparatus including a light source which irradiates light of a specific wavelength constantly or at a specific blinking frequency to a living body, and a controller which controls a blinking frequency of the light source, a step of operating the apparatus to control the blinking frequency of the light source to be 0 Hz or to be in a range of 30 Hz to 75 Hz by the controller, and a step of operating the apparatus such that the light source irradiates light of a wavelength in a range of 350 nm to 400 nm to a living body.

Furthermore, one aspect of the present invention is related to an instrument for treatment and/or prophylaxis of a disorder by light stimulation, and the instrument may include a glass, a spectacle lens, or a contact lens which allows violet light to pass through. By using such instrument, it is possible to have a preferable effect on a biological function such as survival, proliferation, or differentiation of oligodendrocyte precursor cells or oligodendrocytes, myelinogenesis, activation of nerves, and activation of immunity. By using such instrument, treatment and/or prophylaxis of the disorder may be possible.

### [Examples]

### Example 1

### (Light irradiation on mouse)

A mouse cage was placed in a light exposure device (New Opto, Kanagawa, Japan) having LEDs (white: NF2W757GT-F1, purple: NSSU123, Nichia Corporation, Tokushima) attached to an upper portion of a box. Mice of a control group were reared in white light (WL) from 8 am to 8 pm in a cycle of 12-hour light period / 12-hour dark period. Mice of an experimental group as well, were exposed to white light under the same conditions, except for an exposure to violet light (VL). In the experimental group, mice were exposed to violet light every day from 10 am to 12 am for 16 weeks. For violet light, light of a wavelength in a range of 360 nm to 400 nm (peak wavelength 375 nm) was used, and was irradiated constantly (blinking frequency of 0 Hz) in a range of irradiance of 0.5 µW/cm² to 1000 µW/cm².

### (Tissue conditioning)

After irradiating light for 16 weeks, 85-week-old aged mice were euthanized by cervical dislocation, and an entire brain of each mouse was extracted. Tissues were divided into two halves, and one half was used for immunostaining, while the remaining half was used for analysis of gene expression. All tissues were quick-frozen by liquid nitrogen, and were preserved at -80 °C until the analysis was carried out.

### (Total RNA extraction)

Hippocampal tissues were homogenized in QIAzol lysis reagent (Qiagen, Germany), and a total RNA was extracted by using RNeasy mini kit (Qiagen, Germany). Mass and quantity of all RNA samples were determined by using Nanodrop RNA Nano kit (2100 Agilent Bioanalyzer Technologies, CA, USA). For the analysis of gene expression, only samples with RIN (RNA integrity number) higher than 8.0 were used.

### (Quantitative real-time PCR)

The total RNA was isolated from each tissue by QIAzol lysis reagent (Qiagne, Germany) as mentioned above, and a concentration was measured by Nanodrop Onec (Thermo Scientific). The total RNA of 500 ng was used for cDNA synthesis by a reverse transcription oxygen RNA kit (Applied Bio-Systems, Japan). Quantitative real-time PCR was carried out according to a protocol mentioned in the past documents. To explain briefly, a PCR reaction in which SYBR Green is used was carried out under the following conditions using Quant studio 5: 40 cycles of, two minutes at 50 °C, followed by 10 seconds at 95 °C and one minute at 60 °C. A value of each transcript was calculated as an average for two samples at all experimental conditions. The values were normalized with GAPDH as an internal control. Data was analyzed by comparing cycle threshold values (Ct) of different groups by 2-Δ (ΔCt) method.

### (Change in gene expression of hippocampus by VL irradiation)

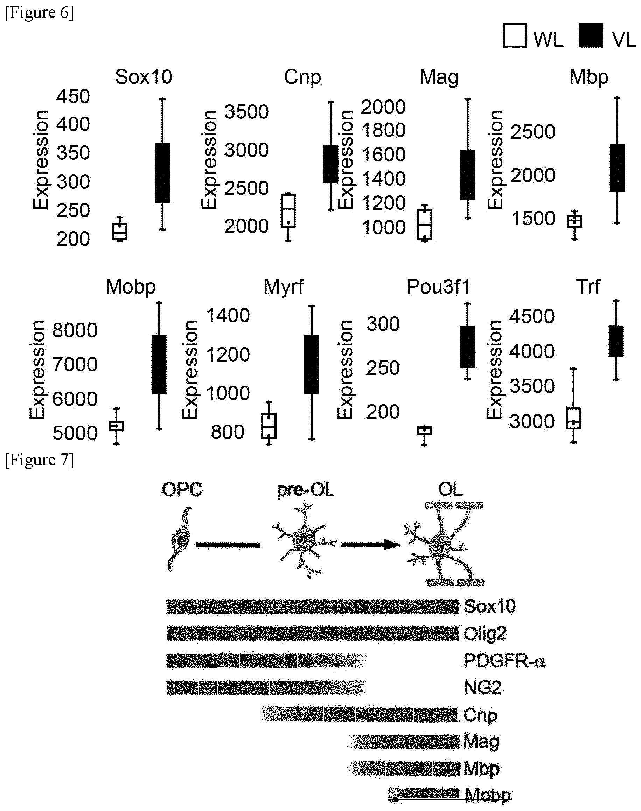

For evaluating an effect of violet light (VL) stimulation on gene expression profile of a brain, RNA-seq analysis was carried out for the hippocampus after the aged mice were exposed to VL. As a result, 404 DEGs (differentially expressed genes) including 231 up-regulated genes and 173 down-regulated genes were identified. Gene ontology (GO) analysis was carried out, and as a result of classifying the DEG into up-regulated genes and down-regulated genes, it was revealed that the VL stimulation increases the expression of genes involved in neural circuit formation such as covering of neuron, axon covering, and myelinogenesis. It is noteworthy that the development of glia cells, particularly, differentiation of oligodendrocytes, is included in a cluster of up-regulated genes. Thus, the aged-mice exposed to VL indicate up-regulation of gene associated with the differentiation of oligodendrocytes. Specifically, while the genes up-regulated by VL play a significant role in differentiation from pre-oligodendrocytes to matured oligodendrocytes, genes involved in myelin maturation such as Sox10, Cnp, Mag, Mbp, and Mobp are included in this (Fig. 6 and Fig. 7). Furthermore, VL increased an expression of Myrf which is a known Sox10 cofactor that induces oligodendrocyte differentiation genes. VL also induces other oligodendrocyte-associated genes such as Pou3f1 and transferon (Trf) (Fig. 6). These data indicate that VL stimulation changes the gene expression involved in glia differentiation, as a result of which myelinogenesis is accelerated, thereby indicating that the treatment or prophylaxis of the demyelination disorder is possible.

### Example 2

MBP (myelin basic protein) which is a biomarker of matured oligodendrocytes was detected from cerebral cortex acquired from 88-week-old aged mice to which violet light was irradiated for 18 weeks similarly as in Example 1, and was stained by DAPI which stains DNA.

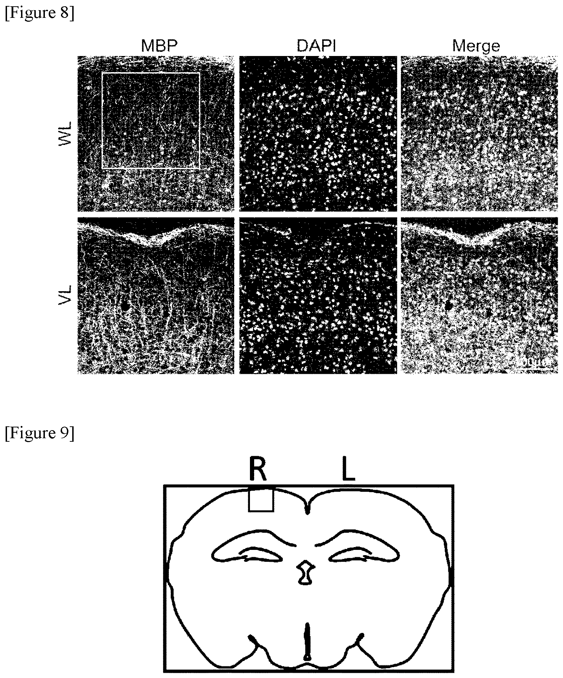

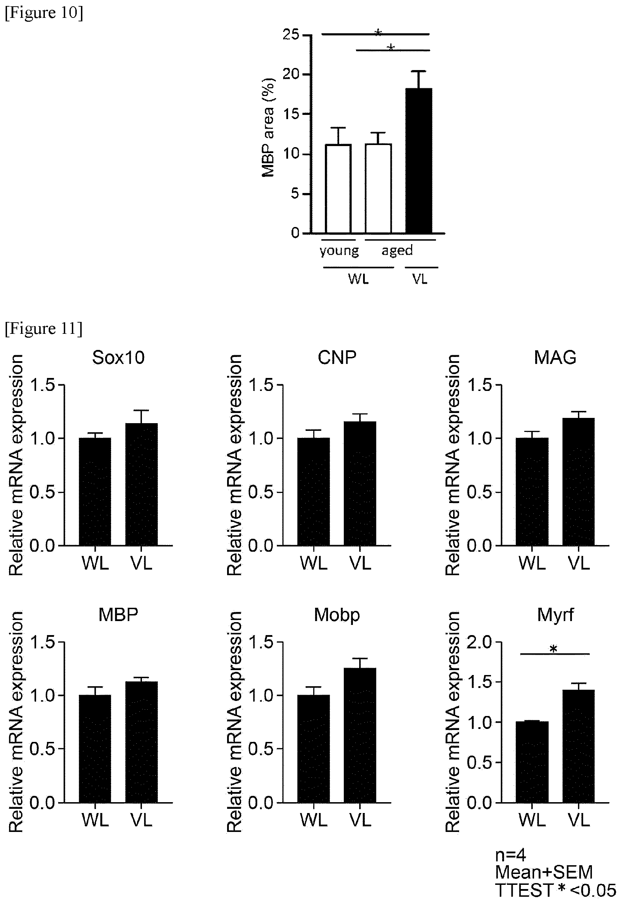

Results are shown in Fig. 8 to Fig. 10. In Fig. 8, VL shows results for mice of an experimental group to which violet light was irradiated. WL shows results for mice of a control group to which violet light was not irradiated. Fig. 9 shows a site of the cerebral cortex acquired. Fig. 10 is a graph in which the mice of the experimental group and the mice of the control group are compared in an area in which MBP is expressed. The number of mice used for the experiment was three, and 3 to 4 sites for each mouse were examined. In Fig. 10, 'Aged' denotes 88-week-old and 'young' denotes 13-week-old. Mean+SEM was * < 0.05 at One-way - ANOVA +post hoc (Tukey).

As shown in Fig. 8 and Fig. 10, by rearing in an environment of violet light irradiation, an area in which MBP is expressed in the cerebral cortex increased, and it was revealed that the matured oligodendrocytes increase in the cerebral cortex of the aged mouse. From this, it is revealed that the method of the present invention is applicable to treatment and/or prophylaxis of a neurogenerative disorder such as a recovery from Alzheimer disease, multiple sclerosis, amyotrophic lateral sclerosis (ALS), and spinal damage. Moreover, it is revealed that the method of the present invention is applicable to treatment and/or prophylaxis of brain tumor (glioma). Furthermore, it is revealed that the method of the present invention is applicable to treatment and/or prophylaxis of pain, numbness, leaking of urine, feeling light in one's head, failing vision, and fatigue associated with myelinogenesis.

### Example 3

Violet light was irradiated for a short period of one week similarly as in Example 1 to 8-week-old young mice, and by using tissues of hippocampus of brain, the expression of genes SOX10, CNP, MAG, MBP, Mobp, and Myrf which are biomarkers of oligodendrocytes was verified by quantitative PCR. Mice used were C57BL/6j. The number of mice of the experimental group was four, and these four mice were exposed to violet light every day from 10 am to 12 am. The number of mice of the control group was four, and these four mice were reared in white light (WL) from 8 am to 8 pm in a cycle of 12-hour light period / 12-hour dark period.

Results are shown in Fig. 11. In Fig. 11, VL indicates results for the mice of the experimental group to which violet light was irradiated. WL indicates results for the mice of the control group to which violet light was not irradiated.

As shown in Fig. 11, for the mice of the experimental group, the expression of genes SOX10, CNP, MAG, MBP, Mobp, and Myrf in the tissues of brain cortex had increased as compared to that for the mice of the control group. Thus, it was revealed that, in a young state, activation and differentiation of oligodendrocytes can be induced adequately in a short-term violet light. From this, it is revealed that the method of the present invention can be applied to treatment and/or prophylaxis of a disorder such as various disorders of central nervous system and brain tumor, and to the healthcare improvement.

### Example 4

In a social defeat stress model, after irradiating violet light for 10 days similarly as in Example 1 to depression-induced experimental animals (C57B16/J), expression of c-Fos, MBP, CC1, and Iba1 in nucleus accumbens of brain was verified.

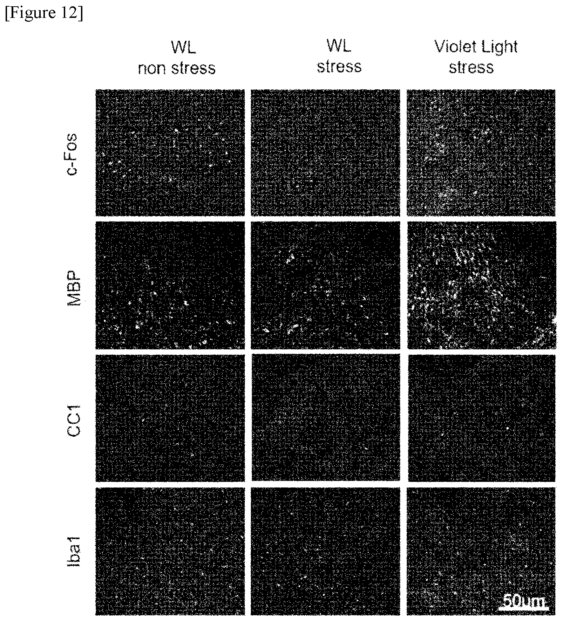

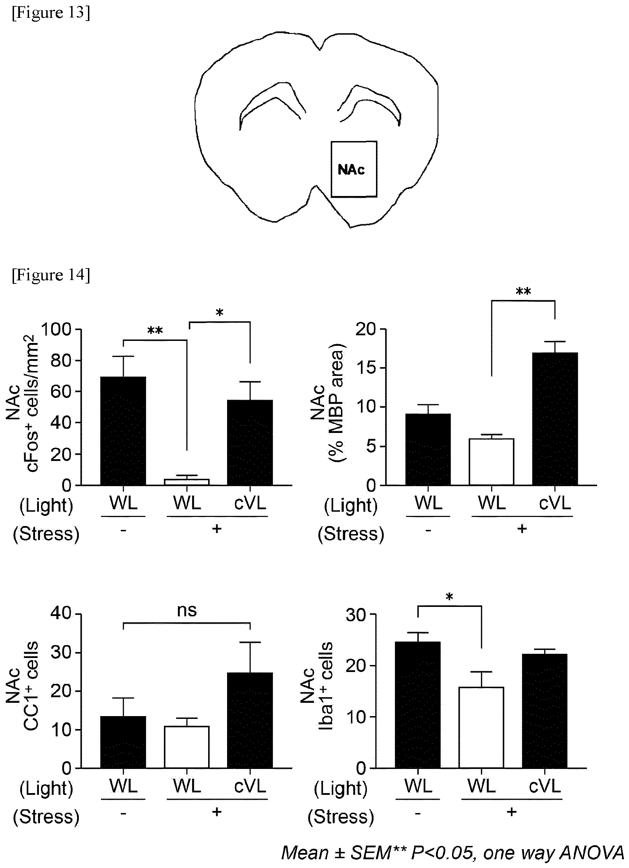

Results are shown in Fig. 12 to Fig. 14. In Fig. 12, Violet Light indicates results for mice of an experimental group to which violet light was irradiated. WL indicates results for mice of a control group to which violet light was not irradiated. In Fig. 12, 'stress' indicates that depression was induced. Whereas, 'non stress' indicates that depression is not induced. Fig. 13 shows a site of nucleus accumbens of brain. Fig. 14 are graphs in which results of photographs in Fig. 12 are quantified. In Fig. 14, WL indicates results for mice of the control group to which violet light was not irradiated. Whereas, cVL indicates results for mice of the experimental group to which violet light was irradiated. Here, 'stress' indicates as to whether depression was induced or not.

As shown in Fig. 12 and Fig. 14, for the mice of the experimental group to which violet light was irradiated, in nucleus accumbens of brain, CC1 and MBP which are matured oligodendrocyte maker had increased, the nerve was activated (c-Fos), and microglial cells which are immunocytes of brain had increased (Iba1). From this, it was revealed that violet light restores oligodendrocytes decreased due to stress, and activates nerves and immunity. Consequently, it is revealed that the method of the present invention can be applied to treatment and/or prophylaxis of a neurogenerative disorder and tumor, and can be applied to the healthcare improvement.

### Example 5

Except for changing the site of brain from nucleus accumbens to prefrontal cortex, an experiment similar to that in Example 4 was carried out.

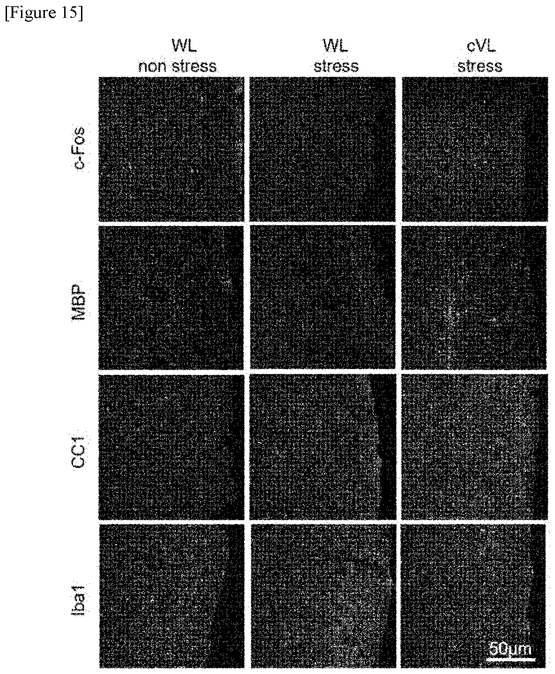

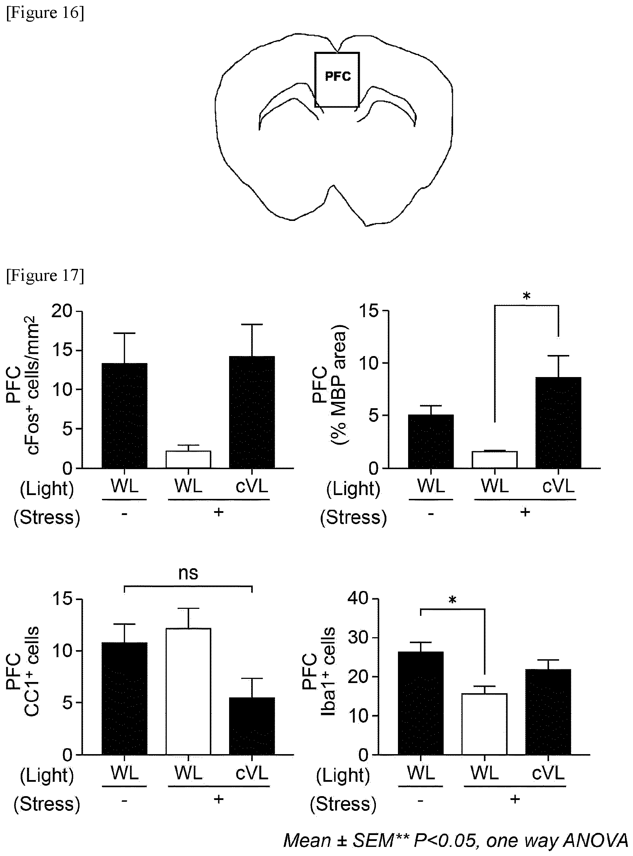

Results are shown in Fig. 15 to Fig. 17. Fig. 16 shows a site of prefrontal cortex of brain.

As shown in Fig. 15 and Fig. 17, for mice of the experimental group to which violet light was irradiated, in the prefrontal cortex of brain, CC1 and MBP which are matured oligodendrocyte makers had increased, the nerve was activated (c-Fos), and microglial cells which are immunocytes of brain had increased (Iba1). From this, it was revealed that violet light restores microglial cells and oligodendrocytes decreased due to stress, and activates nerves and immunity. Consequently, it is revealed that the method of the present invention can be applied to treatment and/or prophylaxis of neurogenerative disorder and tumor, and can be applied to the healthcare improvement.

### Example 6

In a social defeat stress model, violet light was irradiated for 10 days similarly as in Example 1 to experimental animals having no depression induced (C57B16/J) and to depression-induced mice. Gene expression of fibroblast growth factor (FGF) was verified in prefrontal cortex of brain. FGF1 and FGF22 are known as significant factors in myelinogenesis and differentiation of oligodendrocytes.

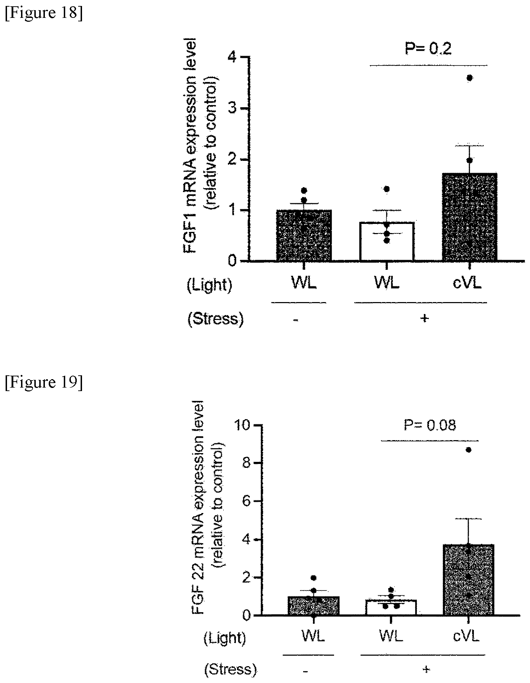

Results are shown in Fig. 18 to Fig. 19. In Fig. 18 and Fig. 19, WL indicates results for mice of a control group to which violet light was not irradiated. Whereas, cVL indicates results for mice of an experimental group to which violet light was irradiated. Here, 'stress' indicates as to whether depression was induced due to social defeat stress model or not.

As shown in Fig. 18 and Fig. 19, it was revealed that by violet light irradiation for 10 days, the gene expression of FGF1 and FGF22 increase in the prefrontal cortex of brain. FGF1 and FGF22 being involved widely in inflammation suppression, repair, and proliferation of cells, it is revealed that the method of the present invention can be used for various applications.

### Example 7

Violet light was irradiated (constantly) for four weeks to 8 to 10-week-old young mice (C57BL/6j), and expression of FGF21 mRNA in hippocampus of left brain and right brain was verified.

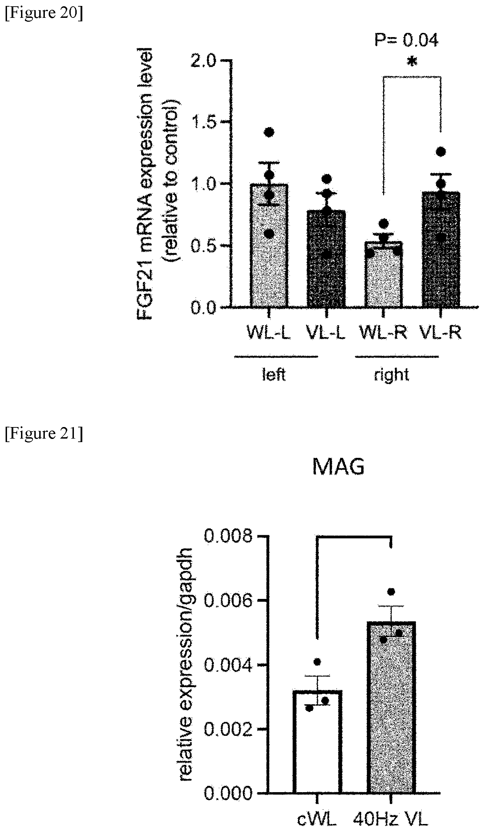

Results are shown in Fig. 20. WL indicates a control group to which white light of 50 lux was irradiated from 8 am to 8 pm. VL indicates an experimental group to which violet light was irradiated from 8 am to 11 am in addition to irradiating white light of 50 lux from 8 am to 8 pm. The number of WL is four and the number of VL is four.

As shown in Fig. 20, the expression of FGF21 was induced in hippocampus of right brain. FGF21 has been known to induce differentiation of oligodendrocytes. Moreover, FGF21 has also been reported to improve cognitive function in brain. From this, it is revealed that the method of the present invention induces differentiation of oligodendrocytes, and can be applied for improvement of the cognitive function of brain.

### Example 8

Except for irradiating violet light at a blinking frequency of 40 Hz instead of irradiating violet light constantly (blinking frequency of 0 Hz), the experiment was carried out similarly as in Example 1 to Example 7.

### Example 9

By using an animal model for Alzheimer disease of P301S mutant human tow gene-induced mice, an experiment similar to these examples except that violet light of 40 Hz was irradiated was carried out, and expression of MAG (myelin associated glycoprotein) of myelinating gene was verified.

Results are shown in Fig. 21. In Fig. 21, 40 Hz VL indicates an experimental group to which violet light of 40 Hz was irradiated. cWL indicates a control group.

As shown in Fig. 21, by irradiation of violet light of 40 Hz, expression of MAG (myelin associated glycoprotein) had increased. From this, it is revealed that irradiation of violet light of 40 Hz accelerates differentiation of oligodendrocytes.

All publications, applications, standards, and patents mentioned in the present specification are herein incorporated by reference in its entirety, and in a case of contradictory terminology, the present specification will take precedence. Scope of the present invention is not to be restricted by specific embodiments described in the present specification. In fact, in addition to the description in the present specification, various modifications of the present invention will be apparent to the person skilled in the art from the abovementioned description and the accompanying drawings. Such amendments are intended to fall within the scope of the appended patent claim. Some or all of the embodiments may also be described as the following supplementary notes, and the disclosure of the present application is not restricted to the following supplementary notes.

### (Supplementary Note 1)

A method of treatment and/or prophylaxis of a disorder in a subject requiring treatment and/or prophylaxis, comprising:
irradiating light of a specific wavelength to the subject,
wherein the disorder is selected from a group consisting of demyelination disorder, neuro-degenerative disorder, tumor, central neurological disorder, and symptoms associated with myelinogenesis.

### (Supplementary Note 2)

The method according to supplementary note 1, comprising:
treating and/or preventing a disorder in the subject by promoting regeneration or an increase in myelin in at least one area of the central nervous system of the subject by irradiating light of a specific wavelength to the subject,
wherein the disorder is selected from a group consisting of demyelination disorder, neuro-degenerative disorder, tumor, central neurological disorder, and symptoms associated with myelinogenesis.

### (Supplementary Note 3)

A method of treatment and/or prophylaxis of a disorder in a subject requiring treatment and/or prophylaxis, comprising:
treating and/or preventing a disorder in the subject by promoting regeneration or an increase in myelin in at least one area of a central nervous system of the subject by irradiating light of a specific wavelength to the subject by controlling a light irradiating apparatus,
wherein the disorder is selected from a group consisting of demyelination disorder, neuro-degenerative disorder, tumor, central neurological disorder, and symptoms associated with myelinogenesis.

### (Supplementary Note 4)

The method according to supplementary note 3, wherein the light irradiating apparatus includes a light source which irradiates light of a specific wavelength constantly or at a specific blinking frequency to a living body, and a controller which controls the blinking frequency of the light source.

### (Supplementary Note 5)

The method according to any one of supplementary notes 1 to 4, wherein the demyelination disorder is selected from a group consisting of multiple sclerosis, neuromyelitis optica (Devic's syndrome), concentric sclerosis (Balo disease), acute disseminated encephalomyelitis (ADEM), inflammatory diffuse sclerosis (Schilder disease), subacute sclerosing panencephalitis (SSPE), progressive multifocal leukoencephalopathy (PML), hypoxic encephalopathy, central pontine myelinolysis, vitamin B12 deficiency, Guillain-Barre syndrome, and Binswanger disease.

### (Supplementary Note 6)

The method according to any one of supplementary notes 1 to 5, wherein the neurogenerative disorder is selected from a group consisting of amyotrophic lateral sclerosis (ALS), Alzheimer disease, and spinal damage.

### (Supplementary Note 7)

The method according to any one of supplementary notes 1 to 6, wherein the tumor is brain tumor (glioma).

### (Supplementary Note 8)

The method according to any one of supplementary notes 1 to 7, wherein the central neurological disorder is cerebral infarction.

### (Supplementary Note 9)

The method according to any one of supplementary notes 1 to 8, wherein the symptom associated with myelinogenesis is selected from a group consisting of pain, numbness, leaking of urine, feeling light in one's head, failing vision, and fatigue.

### (Supplementary Note 10)

A method for promoting regeneration or an increase in myelin in at least one area of a central nervous system of a subject requiring treatment and/or prophylaxis, comprising:
promoting regeneration or an increase in myelin in at least one area of the central nervous system of the subject by irradiating light of a specific wavelength to the subject.

### (Supplementary Note 11)

A method for promoting survival, proliferation, or differentiation of oligodendrocyte precursor cells or oligodendrocytes in at least one area of a central nervous system of a subject requiring treatment and/or prophylaxis, comprising:
promoting survival, proliferation, and differentiation of oligodendrocyte precursor cells or oligodendrocytes in at least one area of the central nervous system of the subject requiring treatment and/or prophylaxis by irradiating light of a specific wavelength to the subject.

### (Supplementary Note 12)

The method according to any one of supplementary notes 1 to 11, wherein the subject is either being administered with or was administered with at least one medication selected from a group consisting of steroids, glatiramer acetate, fingolimod hydrochloride, Siponimod fumaric acid, dimethyl fumarate, interferon β, natalizumab, and ofatumumab.

### (Supplementary Note 13)

The method according to any one of supplementary notes 1 to 12, comprising:
increasing expression of at least one gene selected from a group consisting of Sox10, Cnp, Mag, Mbp, Mobp, Myrf, Pou3f1, and Trf in at least one area of the central nervous system.

### (Supplementary Note 14)

The method according to any one of supplementary notes 1 to 13, wherein the light is violet light.

### (Supplementary Note 15)

The method according to any one of supplementary notes 1 to 14, wherein the specific wavelength includes a range of 350 nm to 400 nm.

### (Supplementary Note 16)

The method according to supplementary note 15, wherein the specific wavelength includes a wavelength of approximately 380 nm.

### (Supplementary Note 17)

The method according to any one of supplementary notes 1 to 16, wherein the blinking frequency is either 0 Hz or in a range of 30 Hz to 70 Hz.

### (Supplementary Note 18)

The method according to supplementary note 17, wherein the blinking frequency is either 0 Hz or in a range of 35 Hz to 60 Hz.

### (Supplementary Note 19)

The method according to supplementary note 18, wherein the blinking frequency is either 0 Hz or approximately 40 Hz.

### (Supplementary Note 20)

The method according to any one of supplementary notes 1 to 19, wherein irradiation conditions further include an irradiation time of the light source.

### (Supplementary Note 21)

The method according to any one of supplementary notes 1 to 20, wherein the light source is spectacles with a light source or a spectacle frame with a light source, a desktop light source, a mobile terminal mounted light source, a light source installed nearby or in front of face, a portable light source, an indoor lighting, and a desk lamp.

### (Supplementary Note 22)

The method according to any one of supplementary notes 1 to 21, wherein the subject is a human.

### (Supplementary Note 23)

An apparatus for treatment and/or prophylaxis of a disorder by light stimulation, comprising:
at least one light source which emits light; and
a driving circuit which drives the light source,
wherein the light emitted by the light source is light of a specific wavelength which produces an effect of treating and/or preventing a disorder by being irradiated to a living body, and
the disorder is selected from a group consisting of demyelination disorder, neuro-degenerative disorder, tumor, central neurological disorder, and symptoms associated with myelinogenesis.

### (Supplementary Note 24)

An apparatus for promoting regeneration or an increase in myelin by light stimulation, comprising:
at least one light source which emits light; and
a driving circuit which drives the light source,
wherein the light emitted by the light source is light of a specific wavelength which produces an effect of promoting regeneration or an increase in myelin by being irradiated to a living body.

### (Supplementary Note 25)

An apparatus for promoting survival, proliferation, or differentiation of oligodendrocyte precursor cells or oligodendrocytes by light stimulation, comprising:
at least one light source which emits light; and
a driving circuit which drives the light source,
wherein the light emitted by the light source is light of a specific wavelength which produces an effect of promoting survival, proliferation, or differentiation of oligodendrocyte precursor cells or oligodendrocytes by being irradiated to a living body.

### (Supplementary Note 26)

The apparatus according to any one of supplementary notes 23 to 25, wherein the driving circuit includes at least one processor which is communicably connected to the light source and at least one memory, and at least one memory for storing commands that are executable by the processor.

### (Supplementary Note 27)

A method of operating an apparatus according to any one of claims 23 to 26 including a light source which irradiates light of a specific wavelength constantly or at a specific blinking frequency to a living body and a controller which controls the blinking frequency of the light source, comprising:
controlling the blinking frequency of the light source either to 0 Hz or in a range of 30 Hz to 75 Hz by the controller; and
irradiating light of a wavelength in a range of 350 nm to 400 nm to the living body.

### (Supplementary Note 28)

A computer program which causes an apparatus including a light source which irradiates light of a specific wavelength constantly or at a specific blinking frequency to a living body and a controller which controls the blinking frequency of the light source, to execute a method of operating according to supplementary note 27.

### (Supplementary Note 29)

A method of increasing expression of at least one gene selected from a group consisting of Sox10, Cnp, Mag, Mbp, Mobp, Myrf, Pou3f1, and Trf in at least one area of a central nervous system of a subject.

### (Supplementary Note 30)

A method of treatment and/or prophylaxis of a demyelination disorder in a subject requiring treatment and/or prophylaxis, comprising:
irradiating light of a specific wavelength to the subject; and
administering at least one medication selected from a group consisting of steroids, glatiramer acetate, fingolimod hydrochloride, Siponimod fumaric acid, dimethyl fumarate, interferon β, natalizumab, and ofatumumab.

### (Supplementary Note 31)

An apparatus for treatment and/or prophylaxis of a disorder by light stimulation, comprising:
at least one light source which emits light; and
a driving circuit which drives the light source,
wherein the driving circuit includes at least one processor which is communicably connected to the light source and at least one memory, and at least one memory for storing commands that are executable by the processor, and
the light source is configured to emit light of a specific wavelength which produces an effect of treating and/or preventing a disorder by being irradiated to a living body, and
the disorder is selected from a group consisting of demyelination disorder, neuro-degenerative disorder, tumor, central neurological disorder, and symptoms associated with myelinogenesis.

### (Supplementary Note 32)

An apparatus for promoting regeneration or an increase in myelin by light stimulation, comprising:
at least one light source which emits light; and
a driving circuit which drives the light source,
wherein the driving circuit includes at least one processor which is communicably connected to the light source and at least one memory, and at least one memory for storing commands that are executable by the processor, and
the light source is configured to emit light of a specific wavelength which produces an effect of promoting regeneration or an increase in myelin by being irradiated to a living body.

### (Supplementary Note 33)

An apparatus for promoting survival, proliferation, or differentiation of oligodendrocyte precursor cells or oligodendrocytes by light stimulation, comprising:
at least one light source which emits light; and
a driving circuit which drives the light source,
wherein the driving circuit includes at least one processor which is communicably connected to the light source and at least one memory, and at least one memory for storing commands that are executable by the processor, and
the light source is configured to emit light of a specific wavelength which produces an effect of promoting survival, proliferation, or differentiation of oligodendrocyte precursor cells or oligodendrocytes by being irradiated to a living body.

### (Supplementary Note 34)

A computer-readable medium which is a non-transitory computer-readable medium having commands stored therein, and as the command is executed by a processor, it is capable of executing for an apparatus including a light source which irradiates light of a specific wavelength constantly or at a specific blinking frequency to a living body, and a controller which controls a blinking frequency of the light source:
a step of operating the apparatus to control the blinking frequency of the light source to 0 Hz or in a range of 30 Hz to 75 Hz by the controller, and
a step of operating the apparatus such that the light source irradiates light of a wavelength in a range of 350 nm to 400 nm to a living body.

### (Supplementary Note 35)

An instrument for treatment and/or prophylaxis of a disorder by light stimulation, comprising:
a glass, a spectacle lens, or a contact lens which allows violet light to pass through, wherein
the disorder is selected from a group consisting of demyelination disorder, neuro-degenerative disorder, tumor, central neurological disorder, and symptoms associated with myelinogenesis.

## Claims

1. A method for treating and/or preventing a disorder in a subject in need thereof, comprising, comprising:
irradiating light of a specific wavelength to the subject,
wherein the disorder is selected from a group consisting of demyelination disorder, neuro-degenerative disorder, tumor, central neurological disorder, and symptoms associated with myelinogenesis.

2. The method according to claim 1, comprising:
treating and/or preventing a disorder in the subject by promoting regeneration or an increase in myelin in at least one area of the central nervous system of the subject by irradiating light of a specific wavelength to the subject,
wherein the disorder is selected from a group consisting of demyelination disorder, neuro-degenerative disorder, tumor, central neurological disorder, and symptoms associated with myelinogenesis.

3. A method for treating and/or preventing a disorder in a subject in need thereof, comprising, comprising:
treating and/or preventing a disorder in the subject by promoting regeneration or an increase in myelin in at least one area of a central nervous system of the subject by irradiating light of a specific wavelength to the subject by controlling a light irradiating apparatus,
wherein the disorder is selected from a group consisting of demyelination disorder, neuro-degenerative disorder, tumor, central neurological disorder, and symptoms associated with myelinogenesis.

4. The method according to claim 3, wherein the light irradiating apparatus includes a light source which irradiates light of a specific wavelength constantly or at a specific blinking frequency to a living body, and a controller which controls the blinking frequency of the light source.

5. The method according to any one of claims 1 to 4, wherein the demyelination disorder is selected from a group consisting of multiple sclerosis, neuromyelitis optica (Devic's syndrome), concentric sclerosis (Balo disease), acute disseminated encephalomyelitis (ADEM), inflammatory diffuse sclerosis (Schilder disease), subacute sclerosing panencephalitis (SSPE), progressive multifocal leukoencephalopathy (PML), hypoxic encephalopathy, central pontine myelinolysis, vitamin B12 deficiency, Guillain-Barre syndrome, and Binswanger disease.

6. The method according to any one of claims 1 to 5, wherein the neuro-degenerative disorder is selected from a group consisting of amyotrophic lateral sclerosis (ALS), Alzheimer disease, and spinal damage.

7. The method according to any one of claims 1 to 6, wherein the tumor is brain tumor (glioma).

8. The method according to any one of claims 1 to 7, wherein the central neurological disorder is cerebral infarction.

9. The method according to any one of claims 1 to 8, wherein the symptom associated with myelinogenesis is selected from a group consisting of pain, numbness, leaking of urine, feeling light in one's head, failing vision, and fatigue.

10. A method for promoting regeneration or an increase in myelin in at least one area of a central nervous system of a subject requiring treatment and/or prophylaxis, comprising:
promoting regeneration or increase in myelin in at least one area of the central nervous system of the subject by irradiating light of a specific wavelength to the subject.

11. A method for promoting survival, proliferation, or differentiation of oligodendrocyte precursor cells or oligodendrocytes in at least one area of a central nervous system of a subject requiring treatment and/or prophylaxis, comprising:
promoting survival, proliferation, and differentiation of oligodendrocyte precursor cells or oligodendrocytes in at least one area of the central nervous system of the subject requiring treatment and/or prophylaxis by irradiating light of a specific wavelength to the subject.

12. The method according to any one of claims 1 to 11, wherein the subject is either being administered with or was administered with at least one medication selected from a group consisting of steroids, glatiramer acetate, fingolimod hydrochloride, Siponimod fumaric acid, dimethyl fumarate, interferon β, natalizumab, and ofatumumab.

13. The method according to any one of claims 1 to 12, comprising:
increasing expression of at least one gene selected from a group consisting of Sox10, Cnp, Mag, Mbp, Mobp, Myrf, Pou3f1, and Trf in at least one area of the central nervous system.

14. The method according to any one of claims 1 to 13, wherein the light is violet light.

15. The method according to any one of claims 1 to 14, wherein the specific wavelength includes a range of 350 nm to 400 nm.

16. The method according to claim 15, wherein the specific wavelength includes a wavelength of approximately 380 nm.

17. The method according to any one of claims 1 to 16, wherein the blinking frequency is either 0 Hz or in a range of 30 Hz to 70 Hz.

18. The method according to claim 17, wherein the blinking frequency is either 0 Hz or in a range of 35 Hz to 60 Hz.

19. The method according to claim 18, wherein the blinking frequency is 0 Hz or approximately 40 Hz.

20. The method according to any one of claims 1 to 19, wherein irradiation conditions further include an irradiation time of the light source.

21. The method according to any one of claims 1 to 20, wherein the light source is spectacles with a light source or a spectacle frame with a light source, a desktop light source, a mobile terminal mounted light source, a light source installed nearby or in front of face, a portable light source, an indoor lighting, and a desk lamp.

22. The method according to any one of claims 1 to 21, wherein the subject is a human.

23. An apparatus for treatment and/or prophylaxis of a disorder by light stimulation, comprising:
at least one light source which emits light; and
a driving circuit which drives the light source,
wherein the light emitted by the light source is light of a specific wavelength which produces an effect of treating and/or preventing a disorder by being irradiated to a living body, and
the disorder is selected from a group consisting of demyelination disorder, neuro-degenerative disorder, tumor, central neurological disorder, and symptoms associated with myelinogenesis.

24. An apparatus for promoting regeneration or an increase in myelin by light stimulation, comprising:
at least one light source which emits light; and
a driving circuit which drives the light source,
wherein the light emitted by the light source is light of a specific wavelength which produces an effect of promoting regeneration or an increase in myelin by being irradiated to a living body.

25. An apparatus for promoting survival, proliferation, or differentiation of oligodendrocyte precursor cells or oligodendrocytes by light stimulation, comprising:
at least one light source which emits light; and
a driving circuit which drives the light source,
wherein the light emitted by the light source is light of a specific wavelength which produces an effect of promoting survival, proliferation, or differentiation of oligodendrocyte precursor cells or oligodendrocytes by being irradiated to a living body.

26. The apparatus according to any one of claims 23 to 25, wherein the driving circuit includes at least one processor which is communicably connected to the light source and at least one memory, and at least one memory for storing commands that are executable by the processor.

27. A method of operating an apparatus according to any one of claims 23 to 26 including a light source which irradiates light of a specific wavelength constantly or at a specific blinking frequency to a living body and a controller which controls a blinking frequency of the light source, comprising:
controlling the blinking frequency of the light source either to 0 Hz or in a range of 30 Hz to 75 Hz by the controller; and
irradiating light of a wavelength in a range of 350 nm to 400 nm to the living body.

28. A computer program which causes an apparatus including a light source which irradiates light of a specific wavelength constantly or at a specific blinking frequency to a living body and a controller which controls the blinking frequency of the light source, to execute a method of operating according to claim 27.
